# EUROPEAN PATENT SPECIFICATION

(11) **EP 1 159 022 B1**
(45) Date of publication and mention of the grant of the patent: **05.07.2006**
(21) Application number: 00909505.0
(22) Date of filing: 10.03.2000
(51) Int. Cl.: A61M 15/00

(54) **IMPROVEMENTS RELATING TO AN INHALATION DEVICE**
VERBESSERUNGEN AN EINEM INHALATIONSGERÄT
AMELIORATIONS APPORTEES A UN DISPOSITIF D'INHALATION

(30) Priority: 10.03.1999 GB 9905538
(43) Date of publication of application: 05.12.2001
(60) Divisional application: 06075629.3
(73) Proprietor: GLAXO GROUP LIMITED, Greenford, Middlesex UB6 ONN (GB)
(72) Inventor: DAVIES, Michael Birsha, Glaxo Wellcome plc, Ware, Hertfordshire SG12 0DP (GB)
(74) Representative: Thompson, Clive Beresford
(86) International application number: PCT/GB2000/000919
(87) International publication number: WO 2000/053248

(56) References cited:
- WO-A-96/13161
- US-A- 4 664 107
- US-A- 5 349 945
- US-A- 5 447 151
- US-A- 5 533 505
- US-A- 5 568 807
- US-A- 5 823 183

## Description

The present invention relates to a device for the protection of a dose of medicament in an inhalation device for use in the administration of medicament to a patient.

The use of inhalation devices in the administration of medicaments, for example in bronchodilation therapy, is well known. Such devices generally comprise a body or housing within which a medicament container is located. A mouthpiece (or nozzle) is typically provided, wherein 'in use' the mouthpiece communicates with the medicament container to allow passage of medicament from the source to the mouthpiece and thence, to the patient.

In a typical dispensing operation the body of the device is held by the patient and the mouthpiece (or nozzle) of the inhalation device is placed in the mouth (or nose) of the patient. The patient inhales, thereby causing transfer of medicament from the medicament container to the interior of the body of the patient.

Many inhalers are known where the medicament is stored in a pocket, which is sealed for the purpose of preventing any loss of medicament during transportation or in order to reduce the moisture contamination of the medicament during the life of the inhaler. Examples of such are disclosed in EP0211595, where the medicament is loaded directly into a blister pack comprising a sheet; which may be laminated, of foil or plastics material which acts as a carrier and which is provided with a number of breakable or openable containers called "blisters" incorporating a sheet secured on a first sheet forming a cover or lid. A plunger can then be carried by the lid and arranged to penetrate a container when the lid is moved to its open position. A device disclosed in WO97/25086, comprises a spider having pads, resiliently urged into contact with the pockets of the device, said pads being raised away from the pocket by rotation of the spider prior to the use of the inhaler.

A device disclosed in US5,860,419 comprises medicament metered into blisters. The foil seal is peeled back to expose the medicament such that as the blister approaches the airway it is exposed for use.

However, the problem with these inhalers is that once they are prepared for use, i.e. once the seal of the pocket or "blister" is broken or removed, the medicament is exposed to the atmosphere inside the inhaler. The medicament could be dislodged from the pocket if the inhaler is shaken, dropped, or the user exhales into the device.

Similar problems exist with reservoir inhalers where the dose is metered in use. Examples of reservoir devices include that known as Turbohaler as described in EP0237507 and E0006715, and the device described in WO97/20589. Other inhalers are disclosed in US-A-5 568 807, US-A-5 447 151, US-A-5 349 945 and WO-A-9613161.

Thus according to the present invention there is provided an inhaler according to claim 1 hereof.

Amongst the advantages of the present invention in one or more of its embodiments are included that it provides a reduction in moisture contamination of the medicament, particulate contamination of the dose, loss of the dose if the device is inverted, dropped or shaken, until the point of inhalation by the patient. Air exhaled into the device by the patient does not come into contact with the medicament, thereby reducing contamination of the medicament by moisture or particulates, or the medicament being dislodged from the pocket by the patient exhaling into the device.

It follows that the advantages of the present invention include that it also ensures that the full dose metered is available to the patient in that part of the dose is prevented from being lost such as, for example being lost within the device, if the complete dose is not taken in the first inhalation, as may be the case with the elderly and children.

Preferably the covering means is only open in the presence of airflow through and/or pressure drop across the airway in a first direction after which it returns to its resting position.

The airflow through and/or pressure drop across the airway in the first direction will generally be caused by patient inhalation.

In one embodiment the covering means senses airflow through and/or pressure drop across the airway and responds thereto. Thus the covering means responds to airflow through and/or pressure drop across the airway in a first-direction by opening.

The means for sensing the airflow though and/or pressure change across the airway and/or the means of responding thereto (i.e. opening said covering means and/or covering the dose more effectively) may incorporate electronic means. Alternatively it may incorporate mechanical or electromechanical means.

When airflow through and/or pressure drop across the airway is in a second direction the covering means preferably responds by more effectively protecting the dose. For example it may more effectively protect the dose by being urged into closer contact with the dose or container retaining the dose.

The airflow through and/or pressure change in the second direction may generally be caused by the patient exhaling into the device.

There are various ways in which a dose can be metered. In one manner the metering occurs during the manufacturing process of the device ("pre-metered") and the metered dose is retained or located in discrete units. Alternatively the device contains a reservoir containing medicament and the dose is metered in use.

Metering may be based on volume of medicament or surface area.

For metering based on volume the dose may be metered in use or pre-metered into a container of the required volume. More specifically the container may be the pocket wherein the dose is retained after it has been metered. The pocket may also form a throughhole during operation of the device.

In the dose protector the container retaining the dose may have a surrounding rim. The rim around the pocket is not specifically to retain the dose but preferably acts to create a better contact with the said covering means.

Preferably, the sealing flap is spaced away from the pocket by the airflow from the pocket once the contact with the pocket is broken.

In a further embodiment of the invention, the dose protector additionally comprises a closure mechanism wherein the at least one sealing flap is held in contact with the pecker by a closure means which prevents the contact between the at least one sealing flap and the pocket being broken by airflow through the airway in any direction. This can create a seal as the closure mechanism may correspond to and complement the shape of the rim. This provides an additional safety feature, decreasing the risk that a child could inhale the medicament accidentally. The movement of the closure mechanism could potentially be combined with the opening of the air inlet. The closure means applies a constant direct pressure on to the sealing flap thereby providing a seal with a long shelf life.

Preferably the pocket has a surrounding rim, which forms the contact point between the pocket and the sealing flap thereby providing a low contact area between the flap and the pocket.

Preferably the sealing flap is made of a flexible, resilient material with a memory, preferably a 3 year memory more preferably a greater than 3 year memory, i.e. the sealing flap stays resilient, biased against the pocket and flexible.

Preferably the sealing flap vibrates in the airflow once the contact with the pocket is broken. This has the effect of increasing the fine particle mass. Features which may influence the ability of the sealing flap are the flexibility of the material, preferably the sealing flap is made of highly flexible material for example a thermoset rubber, to allow the vibration to occur as the pressure changes in the device; the resilience of the sealing flap, if the resilience is too high the sealing flap may not open, if too low the sealing flap may not close therefore vibrations would not occur.

Preferably the sealing flap is of equivalent or slightly reduced width relative to the distance between the inside walls of the housing at the base of the walls of the housing where the sealing flap is in contact with the pocket. Preferably the distance between the inside walls of the housing increases as the distance away from the pocket increases preventing the sealing flap being hampered in its movement as it vibrates.

It will be appreciated that the vibrations of the sealing flap can be affected by the length of the sealing flap and its anchor position on the housing relative to the position of the pocket. The height of the airway may also effect the vibration of the sealing flap by influencing the curve of the sealing flap. The curve of the sealing flap can be affected by having a locally thinned area on the sealing flap which acts as a hinge, instead of or as well as being made of a highly flexible material.

The covering means is preferably spaced away from the container retaining the dose to coincide with the airflow through and/or pressure drop across the airway once the contact with the dose or container retaining the dose is broken.

In alternative embodiments of the invention the covering means may vibrate in the airflow through and/or pressure drop across the airway in the first direction.

The housing preferably contains a valve flap such that when the airflow is in a second opposite direction, the airflow exits the housing by means of the valve flap. This arrangement allows the airflow to escape from the housing, to prevent build up of pressure.

The said covering means preferably protects the dose from the patient exhaling into the device, moisture contamination, particulate contamination, loss of the dose (e.g. in the device) or a combination thereof. The inhaler may comprise a body and a mouthpiece.

Preferably the inhaler is a dry powder inhaler. For example it may be a reservoir multidose dry powder inhaler, a pre-metered multidose dry powder inhaler or a unit dose dry powder inhaler.

Preferably, the contact between the at least one sealing flap and the pocket is broken by airflow towards the mouthpiece.

Even more preferably, the contact between the at least one sealing flap and the pocket is not broken by airflow from the meuthpiece through the body.

Preferred embodiments of the inhalation device according to the present invention will now be described with reference to the accompanying drawings in which:
Fig. 1a is a sectional side view of a dose protector according to the present invention wherein the sealing flap is at rest.
Fig. 1b is a sectional side view of a dose protector according to the present invention wherein the airflow is in the direction to activate the sealing flap.
Fig. 2a is a sectional side view of a device as shown in Fig 1a and Fig 1b, wherein the valve flap is at rest.
Fig. 2b is a sectional side view of a device as shown in Fig 1a and Fig 1b, wherein the valve is open.
Fig. 2c is a sectional side view of a device as shown in Fig 1a and Fig 1b wherein the airflow is in the direction to activate the sealing flap.
Fig. 3a is a sectional side view of a device (not in accordance with the invention) as shown in Fig 1a and Fig 1b, wherein the pocket has geometry particularly suitable for a metered dose inhaler.
Fig. 3b is a sectional side view of a device (not in accordance with the invention) as shown in Fig 1a and Fig 1b, wherein the airflow is in the direction to activate the sealing flap.
Fig. 4a is a dose protector with closure mechanism in the closed position.
Fig. 4b is a dose protector with the dosure mechanism in the open position.
Fig. 5 is a sectional side view of a device (not in accordance with the invention) as shown in Fig 1a and Fig 1, with a pocket seal.
Fig. 6 is a sectional side view of a unit dose powder inhaler having a dose protector as shown in Fig 1 a and Fig 1b, where the sealing flap is at rest.
Fig. 7 is a perspective view of a unit dose inhaler having a dose protector shown in Fig 1a and Fig 1b at rest.
Fig. 8 is a perspective view of a moulding for a unit dose inhaler similar to that shown in Fig. 6.
Fig 9 is a different perspective view of a unit dose inhaler similar to that shown in Fig. 6

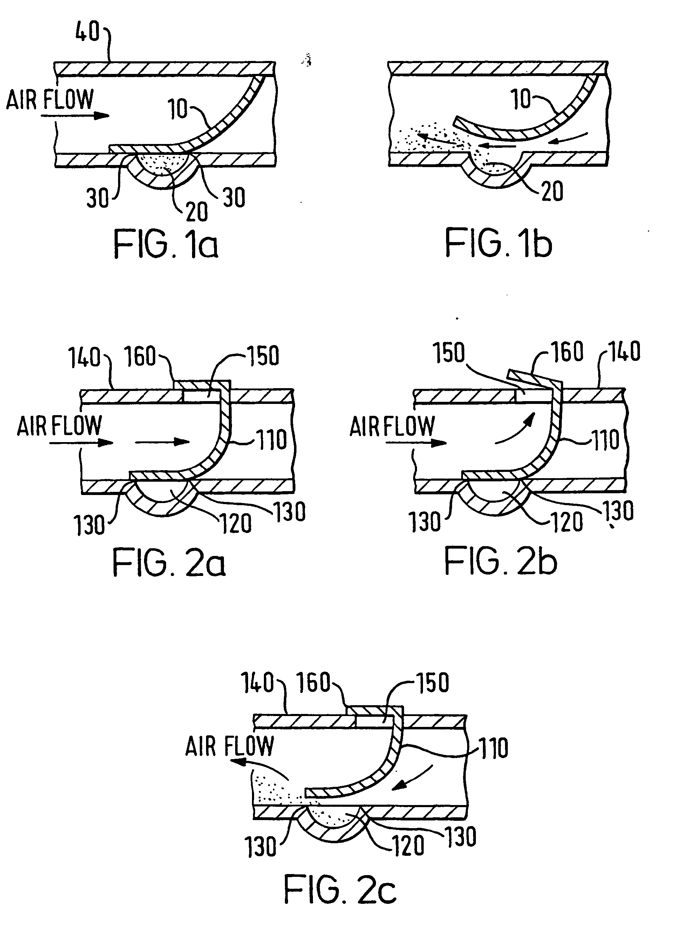

Figures 1a and 1b show a first dose protector comprising a sealing flap 10 and a pocket 20 suitable for containing a dose of medicament. The sealing flap 10 is resiliently biased into contact with the edge of the pocket 30 to reduce moisture contamination and loss of medicament during transportation. The sealing flap is fixedly attached to the body of the housing 40. When air flows towards the sealing flap as shown in Fig. 1 b, such airflow being created by inhalation by the patient, the contact between flap 10 and the edge of the pocket 30 will break, and medicament is entrained in the airflow.

The sealing flap can vibrate to increase the fine particle mass of the medicament.

Fig. 1 a shows that when the air flows towards the sealing flap 10 in the direction shown, the contact between the sealing flap 10 and the edge of the pocket 30 is not broken, thereby maintaining the protection of the dose from moisture and particulate contamination. Such airflow would usually be created by exhalation.

Figures 2a, 2b and 2c show a second aspect of the invention having a hole 150 in the housing 140 so when the airflow is due to exhalation by the patient the valve flap 160 opens to let the moist air out. However when the airflow is in the opposite direction the contact between the sealing flap 110 and the edge of the pocket 130 is broken.

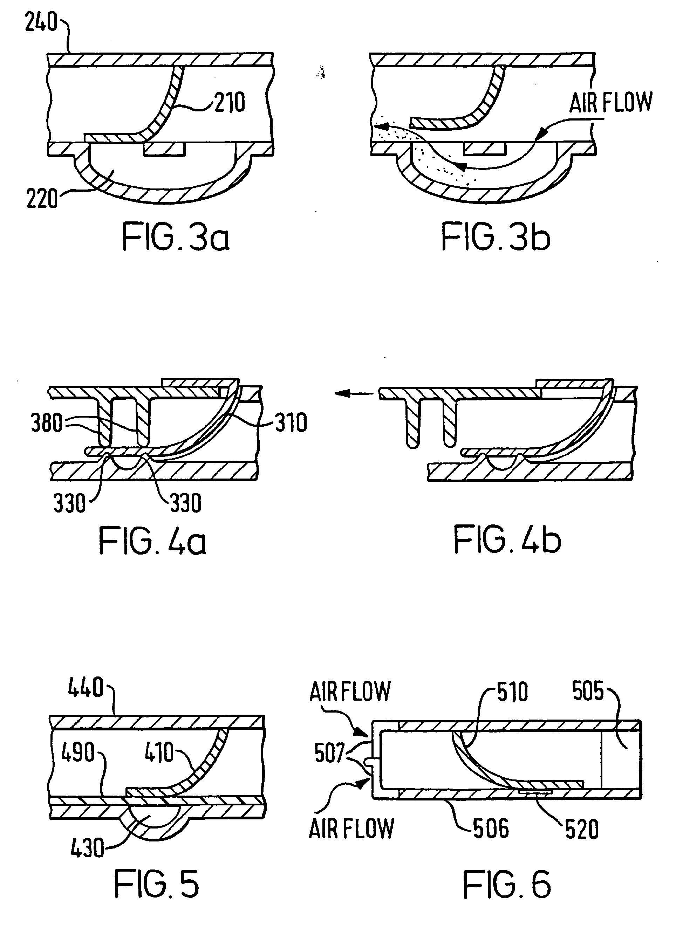

Figures 3a and 3b show a further dose protector having a sealing flap 210 and a pocket 220 having geometry particularly suitable for a metered dose inhaler.

Figures 4a and 4b show the closure mechanism in an open and closed position. The locking means having slidable movement relative to the pocket where in the closed position the legs 380 sit over the edge of the pocket 330 trapping the sealing flap 310 between the legs and the edge of the pocket 330 preventing movement of the sealing flap 310. To open the device, the closure mechanism is slid relative to the pocket so that the legs 380 are not over the rim of the pocket and the sealing flap is not trapped, but free to move when airflow is in the correct direction.

Fig. 5 shows a dose protector additionally having a pocket seal 490, which may be laminated, of foil or plastics material, which is removed to ready the device for use by the patient.

Fig. 6 shows a unit dose powder inhaler having a body 506 a mouthpiece 505 air inlet holes 507 a sealing flap 510 and a pocket 520. When the patient inhales through the mouthpiece 505 air flows into the device through the air inlet holes 507 and the contact between the sealing flap 510 and the pocket 520 is broken. The medicament present in the pocket 520 is entrained in the airflow and carried through the device to be administered to the patient.

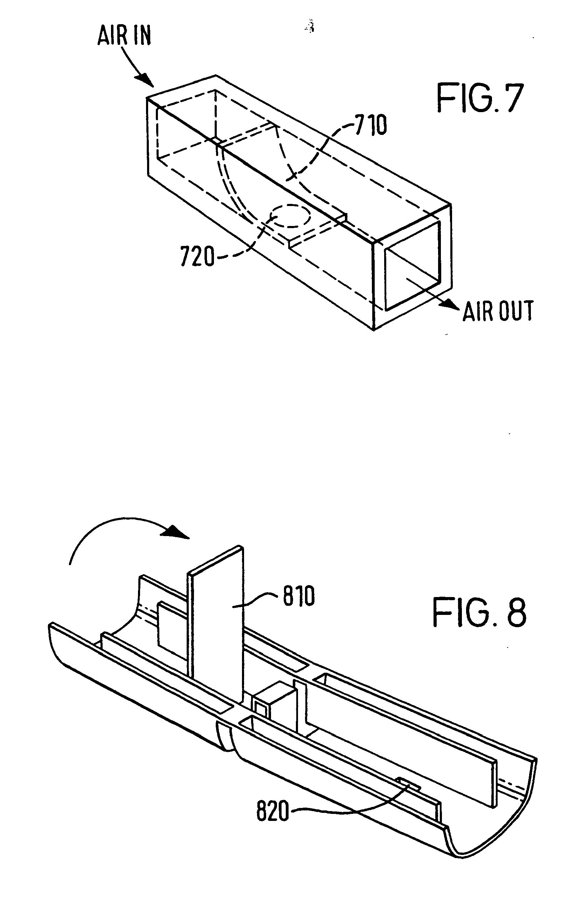

Fig 7 shows a unit dose inhaler wherein pocket 720 containing medicament is covered by sealing flap 710.

Fig 8 shows a one-piece moulding for a unit dose powder inhaler similar to that illustrated in Fig. 6 wherein 820 is the pocket and 810 is a sealing flap which bends into position when one half of the moulding is folded over and closed to form the finished device.

Mouldings as shown in Fig. 8 may be formed in arrays which allows for convenient filling of the pockets with medicament in an automated process.

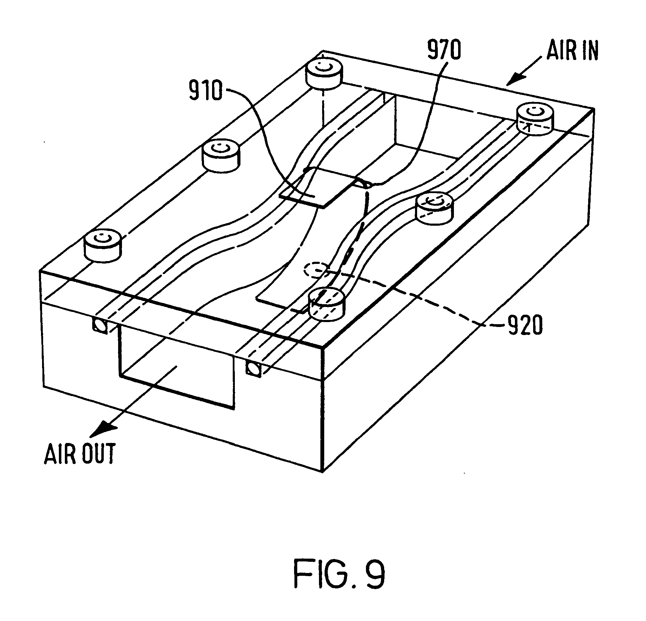

Fig. 9 shows a unit dose inhaler with a pocket containing medicament 920 and a flexible resilient sealing flap 910 located fixedly through a tight fitting slot 970 in the top of the device. The material of the sealing flap is thinned at the slot to improve its positioning.

## Claims

1. An inhaler comprising a housing (40;140;...) defining an airway and a dose protector comprising a dose of medicament retained in a dose container (20;120;...) which is a pocket, and covering means (10;110;...) for said dose which is in the form of at least one sealing flap and which is movable from a covering position, in which it is in biased contact with the pocket and providing a cover for the pocket, and an open position, in which the contact between the at least one sealing flap and the pocket is broken, wherein the at least one sealing flap for the dose is adapted so that it is only able to move from the closed position to the open position in response to airflow through and/or pressure drop across the airway in a first direction but not in a second, opposite direction.

2. An Inhaler as claimed in claim 1 wherein the at least one sealing flap is further adapted to move from the open position to the covering position after removal of airflow through and/or pressure drop across the airway in the first direction.

3. An inhaler as claimed in claim 1 or claim 2 wherein the at least one sealing flap is adapted to move from the covering position to the open position in response to an airflow through and/or pressure drop across the airway which is caused by patient inhalation.

4. An inhaler as claimed in any one of claims 1 to 3 wherein the at least one sealing flap is adapted to sense airflow through and/or pressure drop across the airway and respond thereto.

5. An inhaler as claimed in claim 4 incorporating electronic means for sensing airflow through and/or pressure drop across the airway and/or means of responding thereto.

6. An inhaler as claimed In any preceding claim wherein the at least one sealing flap is adapted to cover the dose more effectively when the airflow through and/or pressure drop across the airway is in the second direction.

7. An inhaler as claimed in any one of claims 1 to 6 wherein the airflow through and/or pressure drop across the airway in the second direction is able to be caused by the patient exhaling.

8. An inhaler as claimed in any one of claims 1 to 7 wherein the dose is metered by volume of medicament or surface area.

9. An inhaler as claimed in claim 8 wherein the dose is metered by volume into the pocket.

10. An inhaler as claimed in any preceding claim wherein the pocket retaining the dose has a surrounding rim (30;130;...).

11. An inhaler as claimed in any preceding claim additionally comprising a closure mechanism having a closure means (380) for holding the at least one sealing flap in contact with the pocket such as to prevent the contact between the at least one sealing flap and the pocket being broken by airflow through the airway in any direction.

12. An inhaler as claimed in any one of the preceding claims
wherein the at least one sealing flap is adapted to vibrate in the airflow once the contact with the pocket is broken.

13. An inhaler as claimed in any one of the preceding claims
wherein the at least one sealing flap is made of thermoset rubber.

14. An inhaler as claimed in any one of the preceding claims wherein the at least one sealing flap is of equivalent or slightly reduced width relative to the distance between the inside walls of the housing at the base of the walls of the housing where the at least one sealing flap is in contact with the pocket.

15. An inhaler as claimed in claim 14 wherein the distance between the inside walls of the housing increases as the distance away from the pocket increases.

16. An inhaler as claimed in any one of claims 1 to 15 wherein in the open position the at least one sealing flap is spaced away from the pocket retaining the dose to coincide with the airflow through and/or pressure drop across the airway in the first direction.

17. An inhaler as claimed in any one of claims 1 to 16 wherein the housing contains a valve flap (160) for enabling the airflow to exit the housing when the airflow is in the second opposite direction.

18. An Inhaler as claimed in any preceding claim wherein the at least one sealing flap is adapted in use to protect the dose from the patient exhaling in the airway, moisture contamination, particulate contamination and loss of the dose or a combination thereof.

19. An inhaler as claimed in any one of the preceding claims in which the dose of medicament is in powder form.

20. An inhaler as claimed in any preceding claim comprising a body and a mouthpiece.

21. An inhaler as claimed in claim 20 wherein the inhaler is a dry powder inhaler.

## Patentansprüche

1. Inhalator, mit einem Gehäuse (40; 140; ...), das einen Luftweg definiert, und einem Dosis-Schutz mit einer Medikamentendosis, die in einem Dosis-Behälter (20; 120; ...) enthalten ist, der eine Tasche ist, und einer Abdeckeinrichtung (10; 110; ...) für die Dosis, die in der Form von zumindest einer Dichtungsklappe ist und die aus einer Abdeckposition, in welcher sie in einem vorgespannten Kontakt mit der Tasche ist und eine Abdeckung für die Tasche vorsieht, und einer offenen Position bewegbar ist, in welcher der Kontakt zwischen der zumindest einen Dichtungsklappe und der Tasche unterbrochen ist, wobei die zumindest eine Dichtungsklappe so angepasst ist, dass sie sich lediglich von der geschlossenen Position in die offene Position als Reaktion auf einen Luftfluss durch und/oder Druckabfall über den Luftweg in eine erste Richtung bewegen kann, nicht aber in eine zweite, entgegengesetzte Richtung.

2. Inhalator nach Anspruch 1, bei dem die zumindest eine Dichtungsklappe ferner angepasst ist, um sich aus der offenen Position in die Abdeckposition zu bewegen, nach der Beseitigung eines Luftflusses durch und/oder Druckabfalls über den Luftweg in der ersten Richtung.

3. Inhalator nach Anspruch 1 oder Anspruch 2, bei dem die zumindest eine Dichtungsklappe angepasst ist, um sich aus der Abdeckposition in die offene Position zu bewegen, als Reaktion auf einen Luftfluss durch und/oder Druckabfalls über den Luftweg, der durch eine Inhalation eines Patienten bewirkt wird.

4. Inhalator nach einem der Ansprüche 1 bis 3, bei dem die zumindest eine Dichtungsklappe angepasst ist, um einen Luftfluss durch und/oder Druckabfall über den Luftweg wahrzunehmen, und darauf zu reagieren.

5. Inhalator nach Anspruch 4, der eine elektronische Einrichtung aufgenommen hat, zum Wahrnehmen eines Luftflusses durch und/oder Druckabfalls über den Luftweg, und/oder eine darauf reagierende Einrichtung.

6. Inhalator nach einem der vorhergehenden Ansprüche, bei dem die zumindest eine Dichtungsklappe angepasst ist, um die Dosis effektiver abzudecken, wenn der Luftfluss durch und/oder Druckabfall über den Luftweg in der zweiten Richtung ist.

7. Inhalator nach einem der Ansprüche 1 bis 6, bei dem der Luftfluss durch und/oder Druckabfall über den Luftweg in der zweiten Richtung durch das Ausatmen eines Patienten bewirkt werden kann.

8. Inhalator nach einem der Ansprüche 1 bis 7, bei dem die Dosis über das Volumen oder den Oberflächenbereich eines Medikaments abgemessen wird.

9. Inhalator nach Anspruch 8, bei dem die Dosis, über das Volumen, in die Tasche abgemessen wird.

10. Inhalator nach einem der vorhergehenden Ansprüche, bei dem die Tasche, die die Dosis enthält, einen umgebenden Rand (30; 130; ...) aufweist.

11. Inhalator nach einem der vorhergehenden Ansprüche, zusätzlich mit einem Schließmechanismus mit einer Schließeinrichtung (380), um die zumindest eine Dichtungsklappe mit der Tasche in Kontakt zu halten, um zu verhindern, dass der Kontakt zwischen der zumindest einen Dichtungsklappe und der Tasche durch einen Luftfluss durch den Luftweg in irgendeiner Richtung unterbrochen wird.

12. Inhalator nach einem der vorhergehenden Ansprüche, bei dem die zumindest eine Dichtungsklappe angepasst ist in dem Luftfluss zu vibrieren, sobald der Kontakt mit der Tasche unterbrochen ist.

13. Inhalator nach einem der vorhergehenden Ansprüche, bei dem die zumindest eine Dichtungsklappe aus wärmeausgehärtetem Gummi hergestellt ist.

14. Inhalator nach einem der vorhergehenden Ansprüche, bei dem die zumindest eine Dichtungsklappe, relativ zu dem Abstand zwischen den Innenwänden des Gehäuses an der Basis der Wände des Gehäuses, wo die zumindest eine Dichtungsklappe mit der Tasche in Kontakt ist, eine ihm entsprechende oder geringfügig verringerte Breite aufweist.

15. Inhalator nach Anspruch 14, bei dem der Abstand zwischen den Innenwänden des Gehäuse zunimmt, während der Abstand weg von der Tasche zunimmt.

16. Inhalator nach einem der Ansprüche 1 bis 15, bei dem in der offenen Position die zumindest eine Dichtungsklappe von der Tasche, die die Dosis enthält, weg beabstandet ist, um mit dem Luftfluss durch und/oder Druckabfall über den Luftweg in der ersten Richtung übereinzustimmen.

17. Inhalator nach einem der Ansprüche 1 bis 16, bei dem das Gehäuse eine Ventilklappe (160) umfasst, um dem Luftfluss zu ermöglichen aus dem Gehäuse auszutreten, wenn der Luftfluss in der zweiten entgegengesetzten Richtung ist.

18. Inhalator nach einem der vorhergehenden Ansprüche, bei dem die zumindest eine Dichtungsklappe angepasst ist bei der Nutzung die Dosis vor dem Patienten, der in den Luftweg ausatmet, Feuchtigkeitskontamination, Partikel-Kontamination und Verlust der Dosis, oder einer Kombination davon, zu schützen.

19. Inhalator nach einem der vorhergehenden Ansprüche, bei dem die Medikamentendosis in Pulverform ist.

20. Inhalator nach einem der vorhergehenden Ansprüche, mit einem Körper und einem Mundstück.

21. Inhalator nach Anspruch 20, wobei der Inhalator ein Trockenpulverinhalator ist.

## Revendications

1. Inhalateur comprenant un boîtier (40 ; 140 ; ...) définissant un passage d'air et un dispositif de protection de la dose comprenant une dose de médicament conservée dans un conteneur de dose (20 ; 120 ; ...) qui est une poche, et un moyen de couverture (10 ; 110 ; ...) de ladite dose qui se présente sous la forme d'au moins un volet de fermeture mobile depuis une position de couverture, dans lequel il se trouve en contact de façon inclinée avec la poche et fournissant une couverture pour la poche, et une position d'ouverture, dans laquelle le contact entre le ou chaque volet de fermeture et la poche est rompu, dans lequel le ou chaque volet de fermeture de la dose est adapté de façon à ce qu'il soit seulement capable de se déplacer depuis la position de fermeture vers la position d'ouverture en réponse au flux d'air et/ou à la chute de pression à travers le passage d'air dans une première direction mais pas dans une seconde, direction opposée.

2. Inhalateur selon la revendication 1 dans lequel le ou chaque volet de fermeture est en outre adapté pour se déplacer depuis la position d'ouverture vers la position de couverture après l'évacuation du flux d'air et/ou de la chute de pression à travers le passage d'air dans la première direction.

3. Inhalateur selon la revendication 1 ou la revendication 2 dans lequel le ou chaque volet de fermeture est adapté pour se déplacer depuis la position de couverture vers la position d'ouverture en réponse à un flux d'air et/ou à une chute de pression à travers le passage d'air provoqué(e) par l'inhalation du patient.

4. Inhalateur selon l'une quelconque des revendications 1 à 3 dans lequel le ou chaque volet de fermeture est adapté pour capter le flux d'air et/ou la chute de pression à travers le passage d'air et y répondre.

5. Inhalateur selon la revendication 4 incorporant un moyen électronique pour capter le flux d'air et/ou la chute de pression à travers le passage d'air et/ou les moyens y répondant.

6. Inhalateur selon l'une quelconque des revendications précédentes dans lequel le ou chaque volet de fermeture est adapté pour couvrir la dose plus efficacement lorsque le flux d'air et/ou la chute de pression à travers le passage d'air se trouvent dans la seconde direction.

7. Inhalateur selon l'une quelconque des revendications 1 à 6 dans lequel le flux d'air et/ou la chute de pression à travers le passage d'air dans la seconde direction sont capables d'être provoqués par l'expiration du patient.

8. Inhalateur selon l'une quelconque des revendications 1 à 7 dans lequel la dose est dosée par volume de médicament ou surface.

9. Inhalateur selon la revendication 8 dans lequel la dose est dosée par volume dans la poche.

10. Inhalateur selon l'une quelconque des revendications précédentes dans lequel la poche conservant la dose comporte un bord périphérique (30 ; 130 ;...) .

11. Inhalateur selon l'une quelconque des revendications précédentes comprenant en outre un mécanisme de fermeture présentant un moyen de fermeture (380) destiné à maintenir le ou chaque volet de fermeture en contact avec la poche de façon à éviter le contact entre le ou chaque volet de fermeture et la poche étant rompue par le flux d'air à travers le passage d'air dans n'importe quelle direction.

12. Inhalateur selon l'une quelconque des revendications précédentes dans lequel le ou chaque volet de fermeture est adapté pour vibrer dans le flux d'air une fois que le contact avec la poche est rompu.

13. Inhalateur selon l'une quelconque des revendications précédentes dans lequel le ou chaque volet de fermeture est fabriqué en caoutchouc thermodurci.

14. Inhalateur selon l'une quelconque des revendications précédentes dans lequel le ou chaque volet de fermeture est de largeur équivalente ou légèrement réduite par rapport à la distance entre les parois internes du boîtier au niveau de la base des parois du boîtier, à l'endroit où le ou chaque volet de fermeture est en contact avec la poche.

15. Inhalateur selon la revendication 14 dans lequel la distance entre les parois internes du boîtier augmente lorsque la distance s'éloignant de la poche augmente.

16. Inhalateur selon l'une quelconque des revendications 1 à 15 dans lequel, dans la position d'ouverture, le ou chaque volet de fermeture est éloigné de la poche conservant la dose pour coïncider avec le flux d'air et/ou la chute de pression à travers le passage d'air dans la première direction.

17. Inhalateur selon l'une quelconque des revendications 1 à 16 dans lequel le boîtier contient un volet de non-retour à battant (160) destiné à permettre au flux d'air de sortir du boîtier lorsque le flux d'air se trouve dans la seconde direction opposée.

18. Inhalateur selon l'une quelconque des revendications précédentes dans lequel, en fonctionnement, le ou chaque volet de fermeture est adapté pour protéger la dose de l'expiration du patient dans le passage d'air, de la contamination par l'humidité, de la contamination particulaire et de la perte de la dose ou d'une combinaison de ceux-ci.

19. Inhalateur selon l'une quelconque des revendications précédentes dans lequel la dose de médicament se présente sous forme de poudre.

20. Inhalateur selon l'une quelconque des revendications précédentes comprenant un corps et un embout buccal.

21. Inhalateur selon la revendication 20 dans lequel l'inhalateur est un inhalateur à poudre sèche.
